# FASCICULE DE BREVET EUROPEEN

(11) **EP 0 799 622 B1**
(45) Date de publication et mention de la délivrance du brevet: **07.08.2002**
(21) Numéro de dépôt: 97400678.5
(22) Date de dépôt: 26.03.1997
(51) Int. Cl.: A61L 2/22, A61L 9/14, A61L 2/24

(54) **Installation automatique pour l'assainissement de locaux tels que sanitaires**
Automatische Vorrichtung zur Sanierung von Sanitärräumen
Automatic plant for sanitizing sanitary rooms

(30) Priorité: 01.04.1996 FR 9604049
(43) Date de publication de la demande: 08.10.1997
(73) Titulaire: SANITEC, 37000 Tours (FR)
(72) Inventeur: Bolze, Bernard, 37150 La Croix En Touraine (FR)
(74) Mandataire: Wagret, Frédéric

(56) Documents cités:
- EP-A- 0 485 682
- EP-A- 0 559 268
- WO-A-90/12600
- FR-A- 2 302 105

## Description

La présente invention concerne l'installation automatique pour l'assainissement d'une ou plusieurs enceintes disposées dans un ou plusieurs locaux, pourvus d'installations sanitaires, et notamment pour collectivités.

On connaît par EP 485.682 une installation automatique se composant d'un appareil central comprenant un tableau de commande, un réservoir de produit liquide d'assainissement pourvu d'un dispositif de compression et des moyens de pulvérisation d'un produit assainissant liquide. Le réservoir est relié aux moyens de pulvérisation par une unique tubulure. Le produit assainissant contenu dans le réservoir et dans ladite tubulure est mis sous faible surpression pour être projeté dans l'enceinte dès l'ouverture des moyens de pulvérisation. Or, la mise sous pression du produit contenu dans le réservoir supprime toute possibilité d'amovibilité de ce dernier et en rend difficile la réalimentation.

Des moyens de dérivation de pression sont alors nécessaires au niveau du réservoir pour permettre de le remplir par l'intermédiaire d'une canalisation spécifique ou d'intervenir sur l'installation pour sa maintenance ou une réparation particulière. Cette installation connue est susceptible d'améliorations.

On connaît, par les documents EPA 0485 682 et WO 90 12 600 des installations pour la décontamination d'enceintes tels que des sanitaires mais dont la conformation ne permet pas l'utilisation d'un réservoir amovible et dont la pulvérisation n'est pas d'une grande efficacité.

Par ailleurs, on connaît par le document FRA 2 302 105 une installation automatique comprenant un module d'alimentation et des modules de pulvérisation reliés par une canalisation unique apte à être mise sous pression.

Cependant, ce type de dispositif connu ne permet pas de contrôler de manière indépendante le module d'alimentation et les modules de pulvérisation. Ce type d'installation connu conduit à une pulvérisation d'une efficacité réduite, peu flexible et au contrôle limité. Dans ce contexte, la présente invention propose une installation simplifiée permettant d'accéder aisément aux différents éléments, de disposer d'un réservoir amovible et d'augmenter l'efficacité de pulvérisation du produit.

A cette fin, selon l'invention, la présente invention concerne une installation automatique pour l'assainissement d'une ou plusieurs enceintes disposées dans un ou plusieurs locaux, tels que des sanitaires, comprenant :
◆ un module d'alimentation comportant un tableau de commande et un réservoir de produit assainissant liquide;
◆ au moins un module de pulvérisation du produit assainissant, comprenant une électrovanne et un pulvérisateur ;
chaque module de pulvérisation étant relié au module d'alimentation par une unique canalisation ;
caractérisée en ce qu'elle comprend des moyens de mise sous pression dudit produit, uniquement dans ladite canalisation, la pression étant sensiblement supérieure à la pression atmosphérique.

De manière avantageuse, l'installation comporte des moyens d'actionnement du module d'alimentation et du module de pulvérisation, aptes à permettre le fonctionnement séparé et autonome de ces derniers.

Les moyens de mise sous pression comprennent une pompe à faible inertie.

Le module d'alimentation comprend un filtre monté à l'envers de manière à créer une bulle d'air dans celui-ci. De préférence, le réservoir est amovible.

Selon une forme de réalisation avantageuse, l'installation comprend des moyens de purge constitués d'une électrovanne et d'un conduit supplémentaire disposé entre la canalisation et le réservoir.

Le module d'alimentation comporte une minuterie susceptible d'arrêter automatiquement la mise sous pression, après un temps pré-réglé, en cas de rupture d'une canalisation, ou si le réservoir est vide.

Le module de pulvérisation comprend une carte électronique permettant de gérer son fonctionnement, ladite carte comportant des éléments de mémoire effaçables, de données telles que la présence d'un utilisateur dans ledit local, le déclenchement et l'interruption de la pulvérisation, la durée de pulvérisation, la quantité de liquide pulvérisé.

Selon une forme de réalisation, le fonctionnement du module de pulvérisation est déclenché par l'actionnement d'un organe mécanique tel qu'un verrou.

Le liquide est mis sous une pression comprise entre 3 et 10 bars, et de préférence d'environ 4,5 bars.

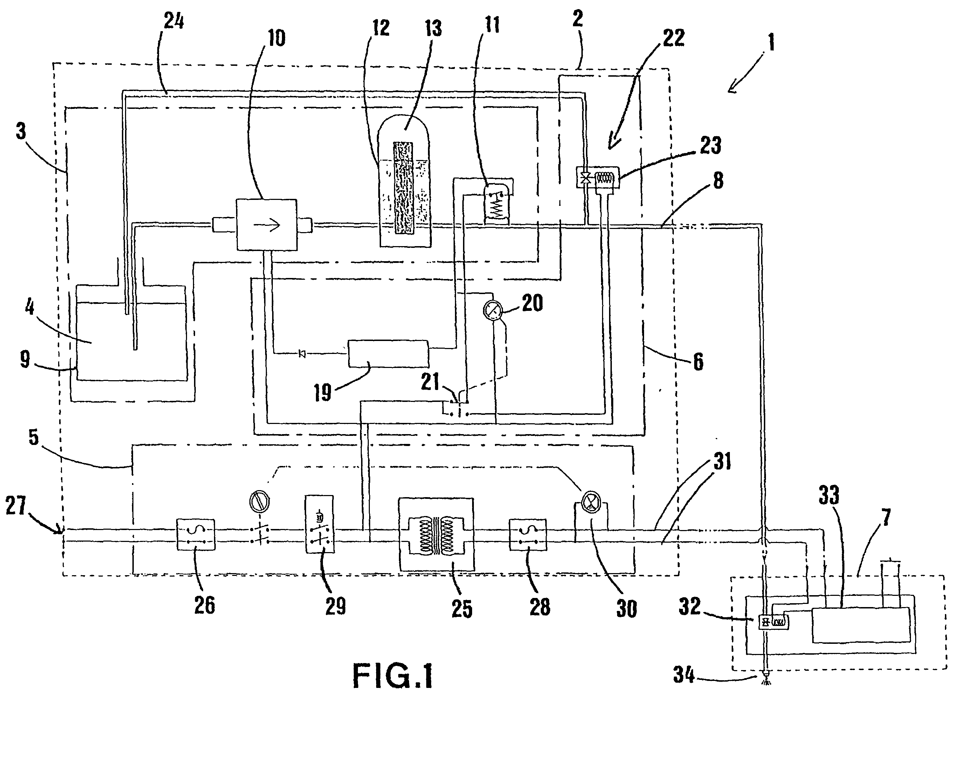

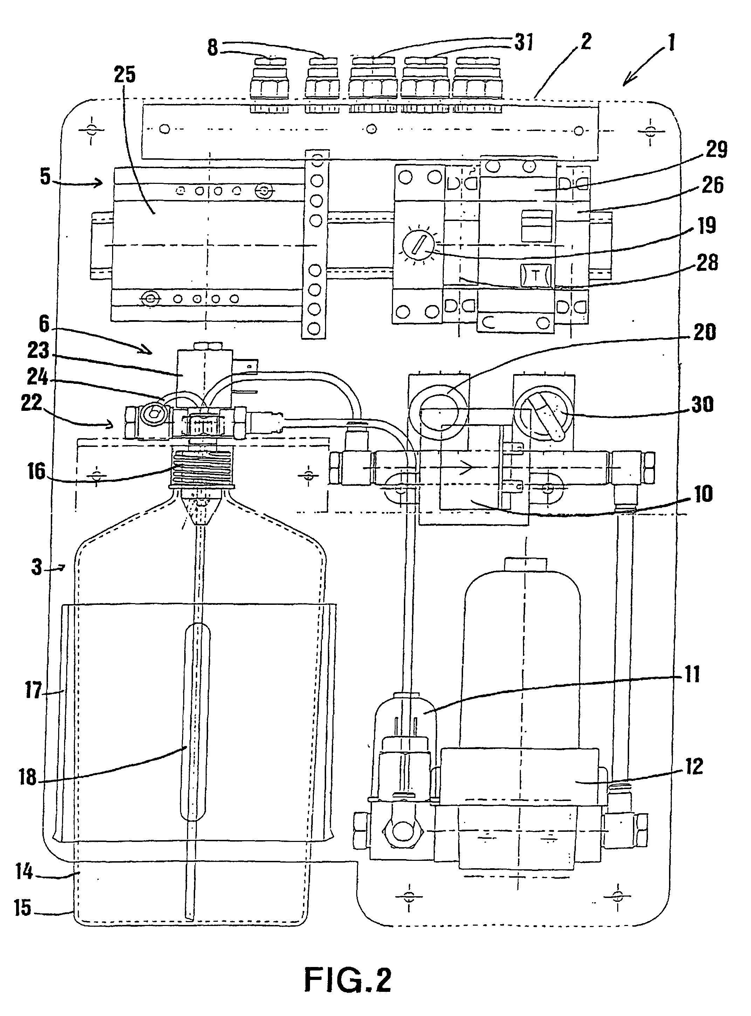

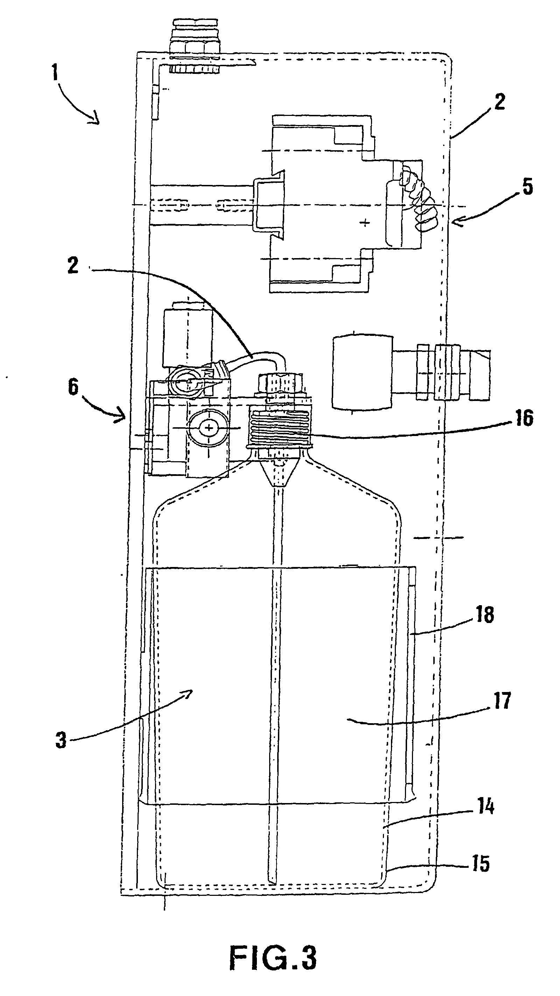

L'invention sera bien comprise à la lumière de la description qui suit se rapportant à un exemple illustratif et non limitatif de l'invention, en référence aux dessins annexés dans lesquels :
◆ la figure 1 représente un schéma synoptique de l'installation selon l'invention incluant un module d'alimentation et un module de pulvérisation ;
◆ la figure 2 représente une vue détaillée en plan, de face, du module d'alimentation de la figure 1 ;
◆ la figure 3 représente une vue détaillée, de côté, de la figure 2.

L'installation 1 automatique d'assainissement d'une ou plusieurs enceintes closes, tels que des sanitaires, comprend, en référence à la figure 1 :
◆ un module d'alimentation 2 destiné à gérer le dispositif 3 d'alimentation en produit 4 assainissant et le dispositif 5 d'alimentation électrique de l'installation, ainsi que les dispositifs 6 de sécurité destinés à protéger celle-ci et permettre une intervention aisée et rapide en cas de panne ;
◆ au moins un module 7 de pulvérisation destiné à projeter le produit liquide assainissant dans l'enceinte ; un module de pulvérisation 7 est prévu par enceinte, dans l'hypothèse d'un local à usage sanitaire, et pourvu d'une pluralité d'enceintes ou d'unités sanitaires ;
◆ une canalisation 8 unique reliant le module 2 d'alimentation et le(s) module(s) 7 de pulvérisation ; un seul module 7 de pulvérisation a été représenté sur la figure 1 pour des raisons de commodité. Dans l'hypothèse où plusieurs modules de pulvérisation sont prévus, ceux-ci sont disposés en série et reliés au module d'alimentation 2 par l'unique canalisation 8 qui, dans ce cas, se ramifie à son extrémité vers les modules de pulvérisation.

Comme montré sur les figures 1 à 3, le module 2 d'alimentation comporte trois parties :
◆ un dispositif 3 d'alimentation en produit assainissant;
◆ un dispositif 5 d'alimentation électrique ; et
◆ un dispositif 6 de sécurité.

Le dispositif 3 d'alimentation en produit 4 assainissant comprend :
◆ un réservoir 9 contenant le produit assainissant ; et
◆ une pompe 10 susceptible d'aspirer le produit 4 depuis le réservoir 9 et de l'introduire dans la canalisation 8 sous une pression supérieure à la pression atmosphérique.

Le produit 4 assainissant est mis sous une pression d'environ 3 à 10 bars, et de préférence de l'ordre de 4,5 bars. La pompe 10 est du type à faible inertie et est donc susceptible de réagir dans un délai d'environ 1/50ème de seconde. Par exemple, une pompe électromagnétique à faible inertie est susceptible de convenir. La pression est régulée par un pressostat 11.

Un filtre 12 est monté à l'envers, à la sortie de la pompe 10. Une bulle d'air 13 est ainsi créée dans sa partie supérieure et permet d'amortir les éventuelles montées soudaines de pression. Le filtre 12 joue le rôle d'un accumulateur hydropneumatique. Dès qu'une pulvérisation est réalisée, la pompe 10 permet de compenser, si cela est nécessaire, la perte de pression dans l'installation. Le produit assainissant contenu dans le réservoir 9 n'est, à aucun moment, mis sous pression; seule la canalisation 8 est mise sous pression. Ainsi, le volume de produit assainissant sous pression est faible, ce qui limite les risques d'incendie en cas de rupture accidentelle de canalisation.

De préférence, le réservoir 9 est amovible et se présente, par exemple, en référence à la figure 2, sous la forme d'un conteneur 14 en matière thermoplastique telle que du polyéthylène. Une enveloppe 15 est apte à recevoir le conteneur 14. Un système de fixation 16 du type à vissage est prévu à l'extrémité supérieure de l'enveloppe 15. De cette manière, le conteneur 14 muni de l'enveloppe 15 est susceptible d'être facilement vissé sur des moyens correspondants du module 2 d'alimentation.

Le conteneur 14 présente à son extrémité supérieure (c'est-à-dire du côté du système de fixation 16) un diamètre supérieur au diamètre du conteneur à son extrémité inférieure. Par exemple, le conteneur présente un diamètre de 12 cm à son extrémité supérieure et de 11 cm à son extrémité inférieure. La différence de diamètre varie d'environ 50 mm à 2 cm.

Un fourreau 17 est fixé sur le module 2 d'alimentation et présente une fente 18 longitudinale comprise dans le plan tangent au fourreau et parallèle au plan du module sur lequel toutes les parties constitutives de celui-ci sont placées. La fente 18 a pour fonction de permettre une lecture directe du niveau d'eau du réservoir 9, c'est-à-dire dans le conteneur 14 dans l'exemple décrit.

Le module 2 d'alimentation se présente par exemple sous la forme d'un boîtier parallélépipédique dont les dimensions sont les suivantes :
◆ la longueur du boîtier est comprise entre 20 et 50 cm et est de préférence de 30,6 cm ;
◆ la hauteur est comprise entre 20 et 80 cm et est de préférence de 43,6 cm ; et
◆ l'épaisseur est d'environ 10 à 30 cm et est de préférence de15,4 cm.

Le dispositif 6 de sécurité comprend :
◆ un système de temporisation 19 disposé en série avec la pompe 10 et relié au pressostat 11 ;
◆ un voyant d'alarme 20 relié à la sortie du système de temporisation 19 et à la pompe 10 ;
◆ un organe mécanique 21 (disjoncteur) du type bouton poussoir relié d'une part au dispositif 5 d'alimentation électrique et d'autre part au pressostat 11 ;
◆ un système de purge 22 relié électriquement à la pompe 10 et au bouton poussoir 21.

Lorsque le réservoir est vide ou que l'une des canalisations de l'installation est rompue, la pression diminue jusqu'à atteindre la pression atmosphérique. Le dispositif 6 le détecte au niveau du pressostat 11 et enclenche le système 19 de temporisation.

Après un temps préréglé au niveau du système 19 par l'utilisateur, entre 10 et 50 seconde environ suivant la taille de l'installation, le fonctionnement de la pompe est interrompu automatiquement. Le voyant 20 d'alarme s'allume pour prévenir l'utilisateur d'un éventuel incident ou du fait que le réservoir est vide. La pompe 10 reste inactive et le voyant 20 reste allumé tant que l'utilisateur n'a pas actionné le bouton 21 poussoir d'alarme.

Le système 22 de purge est constitué par exemple d'une électrovanne 23 de purge permettant d'aspirer l'air ou le produit assainissant de la canalisation 8 et de l'introduire dans un conduit 24 de purge débouchant dans le réservoir 9.

Le système 22 de purge a pour fonction notamment d'abaisser la pression du liquide dans l'installation à la pression atmosphérique pour permettre à l'utilisateur d'intervenir sur celle-ci. Il est également destiné à évacuer l'excédent d'air lors de la mise en marche de l'installation.

Le système 22 de purge est susceptible d'être amorcé au moyen du bouton 21 poussoir d'alarme.

Le dispositif 5 d'alimentation électrique comprend :
◆ un transformateur 25 destiné à alimenter le(s) module(s) 7 de pulvérisation;
◆ des fusibles 26 au niveau du branchement au réseau 27;
◆ des fusibles 28 à la sortie du transformateur 25;
◆ un disjoncteur 29 différentiel éventuellement ajouté pour renforcer la sécurité du dispositif 5;
◆ un voyant 30 de fonctionnement disposé à la sortie du transformateur 25.

Le dispositif 5 d'alimentation électrique est branché sur le réseau 27 (220V - 50 Hz) et est relié soit au système 22 de purge si le bouton 21 poussoir d'alarme est enclenché, soit au pressostat 11 sinon.

Les différentes parties constitutives du dispositif 3 d'alimentation et du dispositif 6 de sécurité sont alimentées en 220 volts. En revanche, pour limiter les risques d'électrocution, le(s) module(s) de pulvérisation est (sont) alimenté(s) en 24 volts. Le transformateur 25 est prévu à cet effet pour alimenter le(s) module(s) de pulvérisation en courant 24 volts alternatif ou continu, à partir du courant délivré par le réseau 27 (220 V).

Le voyant 30 de fonctionnement indique si le(s) module(s) de pulvérisation est (sont) alimenté(s).

Les voyants 20 d'alarme et 30 de fonctionnement présentent une couleur différente. Par exemple, le voyant 20 est jaune et 30 vert.

Des batteries d'accumulateur (non représentées) sont susceptibles d'être incorporées au dispositif 5 d'alimentation pour assurer une alimentation de secours en cas de panne ou d'interruption du réseau.

Chaque module de pulvérisation 7 est alimenté en produit assainissant par l'intermédiaire de la canalisation 8, et alimenté électriquement en 24 volts par le dispositif 5 du module 2 d'alimentation par l'intermédiaire de câbles 31 électriques de connexion.

Comme représenté sur la figure 1, le module 7 de pulvérisation comprend une électrovanne 32 de pulvérisation dont le fonctionnement est géré par une carte 33 électronique.

Une buse 34 permet la projection du produit 4 dans l'enceinte : elle est susceptible d'être intégrée à l'électrovanne 32. La carte 33 permet de déclencher la pulvérisation lorsqu'une condition est remplie, telle que la présence de l'utilisateur dans l'enceinte ou l'ouverture du verrou à la sortie de l'utilisateur. Des temporisations sont susceptibles d'être réglées. Ainsi, par exemple, on peut prévoir un délai de quatre secondes entre l'ouverture du verrou et la pulvérisation pour permettre à l'utilisateur de quitter l'enceinte avant la pulvérisation.

La carte 33 est également apte à interrompre la pulvérisation après un temps déterminé et préréglé au moyen d'un potentiomètre. La durée de pulvérisation est fonction de la quantité nécessaire de produit 4 assainissant pour désinfecter l'enceinte correctement et entièrement. Pour une pression de 4,5 bars, la vitesse de pulvérisation est sensiblement constante et d'environ 1,5 ml par seconde par exemple.

Suivant les dimensions des enceintes, les dimensions et la puissance de chaque module 7 de pulvérisation diffèrent. Les dimensions et la puissance du module 2 d'alimentation sont choisies en fonction du nombre et/ou de la puissance des modules de pulvérisation, ainsi que de la configuration des locaux et de la place allouée à chaque module.

Le fonctionnement du module 2 d'alimentation et du ou des module(s) 7 de pulvérisation est complètement séparé et autonome, ce qui permet de diminuer le nombre de liaisons entre modules et d'en simplifier la structure.

Par ailleurs, les modules d'alimentation et de pulvérisation sont susceptibles d'être installés facilement, sans être gênés par des structures particulières de locaux tels que par exemple éloignés les uns des autres.

## Revendications

1. Installation automatique pour l'assainissement d'une ou plusieurs enceintes disposées dans un ou plusieurs locaux, tels que des sanitaires, comprenant :
- un module d'alimentation comportant un réservoir (9) de produit assainissant liquide (4) ;
- au moins un module (7) de pulvérisation du produit assainissant (4), comprenant un pulvérisateur (34) ;
chaque module (7) de pulvérisation étant relié au module (2) d'alimentation par une unique canalisation (8), ladite installation comprenant des moyens de mise sous pression du produit (4) uniquement dans la canalisation (8), la pression étant sensiblement supérieure à la pression atmosphérique, **caractérisée en ce que** le module d'alimentation comporte un tableau de commande, le module de pulvérisation comprenant une électrovanne (32), et **en ce que** ladite installation comporte des moyens d'actionnement du module d'alimentation (2) et du module de pulvérisation (7) aptes à permettre le fonctionnement séparé et autonome de ces derniers.

2. Installation selon la revendication 1, **caractérisée en ce que** les moyens de mise sous pression comprennent une pompe (10) à faible inertie.

3. Installation selon l'une des revendications précédentes, **caractérisée en ce que** le module d'alimentation (2) comporte une minuterie (19) susceptible d'arrêter automatiquement la mise sous pression après un temps préréglé, en cas de rupture d'une canalisation ou si le réservoir est vide.

4. Installation selon l'une des revendications précédentes, **caractérisée en ce que** le module d'alimentation (2) comprend un filtre (12) monté à l'envers de manière à créer une bulle d'air (13) dans celui-ci.

5. Installation selon l'une des revendications précédentes, **caractérisée en ce qu'**elle comprend des moyens de purge (22) constitués d'une électrovanne (23) et d'un conduit (24) supplémentaire disposé entre la canalisation (8) et le réservoir (9).

6. Installation selon l'une des revendications précédentes, **caractérisée en ce que** le module de pulvérisation comprend une carte électronique (33) permettant de gérer son fonctionnement, ladite carte comportant des éléments de mémoire effaçables de données telle que la présence d'un utilisateur dans le local, le déclenchement et l'interruption de la pulvérisation, la durée de la pulvérisation, la quantité de liquide pulvérisé.

7. Installation selon l'une des revendications précédentes, **caractérisée en ce que** le fonctionnement du module de pulvérisation (7) est déclenché par l'actionnement d'un organe mécanique tel qu'un verrou.

8. Installation selon l'une des revendications précédentes, **caractérisée en ce que** le réservoir (9) est amovible.

9. Installation selon l'une des revendications précédentes, **caractérisée en ce que** le liquide est mis sous une pression comprise entre 3 et 10 bars, et de préférence d'environ 4,5 bars.

## Claims

1. Automatic plant for sanitizing one or more enclosures arranged in one or more premises, such as sanitary rooms, comprising:
- a supply module comprising a tank (9) of liquid sanitizing product (4);
- at least one module (7) for spraying the sanitizing product (4), comprising a sprayer (34);
each spraying module (7) being connected to the supply module (2) via a single pipe (8), said plant comprising means for pressurizing the product (4) solely in the pipe (8), the pressure being substantially higher than atmosphereic pressure, **characterized in that** the supply module comprises a control table, the spraying module comprising an electro-valve (32), and **in that** said plant comprises means for actuating the supply module (2) and the spraying module (7), adapted to allow these latter to function separately and independently.

2. Plant according to Claim 1, **characterized in that** the pressurizing means comprise a low-inertia pump (10).

3. Plant according to one of the preceding Claims, **characterized in that** the supply module (2) comprises a timer (19) capable of automatically stopping the pressurization after a pre-regulated time, in the event of rupture of a pipe or if the tank is empty.

4. Plant according to one of the preceding Claims, **characterized in that** the supply module (2) comprises a filter (12) mounted upside down so as to create an air bubble (13) therein.

5. Plant according to one of the preceding Claims, **characterized in that** it comprises bleed means (22) constituted by an electro-valve (23) and an additional conduit (24) arranged between the pipe (8) and the tank (9).

6. Plant according to one of the preceding Claims, **characterized in that** the spraying module comprises an electronic board (33) for managing its functioning, said board comprising erasable elements for memorizing data such as the presence of a user in the premises, the triggering off and interruption of the spraying, the duration of the spraying, the quantity of liquid sprayed.

7. Plant according to one of the preceding Claims, **characterized in that** functioning of the spraying module (7) is triggered off by the actuation of a mechanical member such as a bolt.

8. Plant according to one of the preceding Claims, **characterized in that** the tank (9) is removable.

9. Plant according to one of the preceding Claims, **characterized in that** the liquid is placed under a pressure included between 3 and 10 bars, and preferably of about 4.5 bars.

## Patentansprüche

1. Automatische Anlage zur Reinigung von einem oder mehreren abgeschlossenen Bereichen, die an einer oder mehreren Stellen vorgesehen sind, wie Nasszellen, die folgendes aufweist:
- ein Zufuhrmodul, das ein Reservoir (9) für flüssiges Reinigungsprodukt (4) aufweist;
- mindestens ein Zerstäubungsmodul (7), das einen Zerstäuber (34) aufweist, für das Reinigungsprodukt (4);
wobei jedes Zerstäubungsmodul (7) über ein einziges Leitungssystem (8) mit dem Zufuhrmodul (2) verbunden ist,
wobei die Anlage Mittel zum Unterdrucksetzen des Produktes (4) nur in dem Leitungssystem (8) aufweist, wobei der Druck wesentlich höher ist als der Atmosphärendruck, **dadurch gekennzeichnet, dass** das Zufuhrmodul eine Bedienungskonsole aufweist, das Zerstäubungsmodul ein Schaltschütz (32) aufweist, und dadurch dass die Anlage Betätigungseinrichtungen für Zufuhrmodul (2) und Zerstäubungsmodul (7) aufweist, die dazu geeignet sind, die getrennte und unabhängige Funktion dieser letzteren zu ermöglichen.

2. Anlage nach Anspruch 1, **dadurch gekennzeichnet dass** die Druckaufbau-Einrichtungen eine trägheitsarme Pumpe (10) aufweisen.

3. Anlage nach einem der vorhergehenden Ansprüche, **dadurch gekennzeichnet, dass** das Zufuhrmodul (2) einen Zeitgeber (19) aufweist, der im Falle einen Bruchs eines Leitungssystems oder wenn das Reservoir leer ist, den Druckaufbau nach einer zuvor festgelegten Zeit automatisch anzuhalten vermag.

4. Anlage nach einem der vorhergehenden Ansprüche, **dadurch gekennzeichnet, dass** das Zufuhrmodul (2) ein Filter (12) aufweist, das verkehrt montiert ist, derart, dass in diesem eine Luftblase (13) erzeugt wird.

5. Anlage nach einem der vorhergehenden Ansprüche, **dadurch gekennzeichnet, dass** sie Entleerungseinrichtungen (22) aufweist, die aus einem Schaltschütz (23) und einer zusätzlichen, zwischen Leitungssystem (8) und Reservoir (9) angeordneten Leitung (24) bestehen.

6. Anlage nach einem der vorhergehenden Ansprüche, **dadurch gekennzeichnet dass** das Zerstäubungsmodul eine elektronische Karte (33) aufweist, durch die sich seine Funktionsweise steuern lässt, wobei die Karte löschbare Speicherelemente für Daten, wie Präsenz eines Anwenders vor Ort, Auslösung und Unterbrechung der Zerstäubung, Dauer der Zerstäubung, Menge der zerstäubten Flüssigkeit, aufweist.

7. Anlage nach einem der vorhergehenden Ansprüche, **dadurch gekennzeichnet, dass** die Funktion des Zerstäubungsmoduls (7) durch Betätigung eines mechanischen Organs, wie eines Riegels, ausgelöst wird.

8. Anlage nach einem der vorhergehenden Ansprüche, **dadurch gekennzeichnet, dass** das Reservoir (9) abnehmbar ist.

9. Anlage nach einem der vorhergehenden Ansprüche, **dadurch gekennzeichnet, dass** die Flüssigkeit unter einem Druck von 3 bis 10 bar, vorzugsweise von etwa 4,5 bar steht.
